# EUROPEAN PATENT APPLICATION

(11) **EP 3 098 635 A1**
(43) Date of publication of application: **30.11.2016**
(21) Application number: 15740578.8
(22) Date of filing: 23.01.2015
(51) Int. Cl.: G02B 6/38, A61B 1/06, G02B 6/42, G02B 23/26

(54) **OPTICAL FIBER CONNECTION ADAPTER AND ENDOSCOPE DEVICE**

(30) Priority: 24.01.2014 JP 2014011415
(71) Applicant: Olympus Corporation, Tokyo 192-8507 (JP)
(72) Inventor: YAJIMA, Hiroyoshi, Tokyo 192-8507 (JP)
(74) Representative: Schicker, Silvia
(86) International application number: PCT/JP2015/000304
(87) International publication number: WO 2015/111413

(57) **Abstract**

An optical fiber connection adapter (10) connects connectors (20a, 20b), which each include a ferrule (23a, 23b) that contains a tip of a single-mode optical fiber (22a, 22b), and guides light from a laser light source from the interior to the exterior of a casing. The adapter (10) includes a housing (11), a sleeve (12) configured so that the ferrules (23a, 23b) are inserted therein and so that the single-mode optical fibers (22a, 22b) of the connectors are optically connected to each other, and a dust protection ring (17) disposed at a casing side of the sleeve (12) and configured to regulate rotation of the sleeve (12) relative to the housing (11) and to shield an interval between the interior of the casing and the interior of the housing (11) when the connector (20a) at the casing side is connected.

## Description

### CROSS-REFERENCE TO RELATED APPLICATION

The present application claims priority from Japanese Application No.2014-011415, filed on January 24, 2014, the entire disclosure of which is incorporated herein by reference.

### TECHNICAL FIELD

This disclosure relates to an optical fiber connection adapter and to an endoscope apparatus using the connection adapter.

### BACKGROUND

An adapter for optical fiber connection is used in order to connect the interior of a casing that contains a laser light source, or of a casing connected to a laser light source, with an external optical fiber. The casing and the optical fiber external to the casing are preferably easily detachable for maintenance, rearrangement, and the like of the apparatus.

In the field of endoscope apparatuses, for example, various endoscopes have been developed. A laser scanning endoscope drives the tip of a scope in a body cavity of an object by vibration, irradiates the examination region while scanning the region with laser light, detects the resulting reflected light or the like, and generates a 2D image. A confocal endoscope uses a confocal technique to obtain a sharp image with high magnification and resolution. An endoscope equipped with a laser light source generates white light with a fluorescent material using the laser light source and irradiates the examination region. In such apparatuses, single-mode optical fiber is used to transmit light. In one example, laser light sources for the three primary colors R, G, and B are disposed within the body of the endoscope, and the light from these lasers is combined with a combiner and guided to the tip of the scope through an optical fiber (for example, see JP 2011-125617 A (PTL 1)).

In general, an endoscope apparatus for observation of living organisms is used by inserting a portion of the scope into a body cavity. Therefore, the scope and the endoscope body containing the light source and the like have a detachable structure to allow cleaning after use. In an endoscope apparatus using lamp light, the lamp is disposed inside the casing of the endoscope body, and the light is, for example, guided to the tip of the scope by a light guide bundle in which light guides with a diameter of slightly less than 100 µm are bundled. Light is transmitted by coupling the light guide bundle between the endoscope body and the scope with an optical fiber connection technique used in optical fiber communication.

The optical fiber connection technique used in optical communication connects optical fiber connectors, which each have a ferrule containing the tip of an optical fiber, using an optical adapter that has a split sleeve. The ferrules in the connected optical fiber connectors are inserted into the split sleeve from either end of the optical fiber adapter, and the cores of the optical fibers abut against each other inside the split sleeve (for example, see JP S59-125706 A (PTL 2), JP 2005-181554 A (PTL 3), and JP 2010-197739 A (PTL 4)). The split sleeve is formed from a hard material, such as zirconia, to position and hold the ferrules together.

### CITATION LIST

### Patent Literature

- PTL 1:: JP 2011-125617 A
- PTL 2:: JP S59-125706 A
- PTL 3:: JP 2005-181554 A
- PTL 4:: JP 2010-197739 A

### SUMMARY

### (Technical problem)

In an endoscope using laser light, however, a single-mode optical fiber for visible light is used. Therefore, the core diameter of the optical fiber becomes extremely small. For example, the core diameter of an optical fiber for optical communication using near-infrared light is approximately 10 µm, whereas the core diameter in an endoscope using laser light is approximately 3.5 µm. When connecting optical fibers by abutting the tips thereof, the variation in connection efficiency increases with each connection. Furthermore, when connecting optical fibers between the interior of the casing and the exterior of the casing, dust or the like stirred up by a cooling fan or the like inside the casing may enter the split sleeve, and the end of the ferrule may be contaminated. If dust or the like attaches between end faces of the optical fibers, the optical fibers may be damaged when abutted, and a fatal malfunction may occur. Therefore, a cleaning operation is necessary each time the scope is attached to the endoscope body, which is inconvenient for the user of the endoscope apparatus.

One of the reasons that the connection efficiency of the optical fibers changes each time the optical fiber connectors are connected is that the split sleeve rotates within the optical fiber adapter, so that the angle ends up changing. Therefore, in PTL 2 to 4, the split sleeve and a portion of the housing of the optical fiber connection adapter are engaged to inhibit rotation of the split sleeve inside the optical fiber adapter. The results of my studies reveal that variation in the connection efficiency of single-mode optical fibers for visible light can also be improved by fixing the angle of the sleeve relative to the housing.

However, when using the optical fiber connection adapters disclosed in PTL 2 to 4 for connection of optical fibers between the interior and the exterior of the casing, dust that enters into the split sleeve from the housing cannot be reduced, even though the connection efficiency can be improved.

Therefore, it would be helpful to provide an optical fiber connection adapter used to connect connectors, which each include a ferrule that contains the tip of a single-mode optical fiber, between the interior of a casing containing or connected to a laser light source and the exterior of the casing, such that a stable optical fiber connection efficiency can be obtained, and dust can be prevented from entering the adapter housing, thus easing the burden of cleaning the fiber end faces.

### (Solution to Problem)

An optical fiber connection adapter according to this disclosure is an optical fiber connection adapter that connects connectors and guides light from a laser light source from an interior to an exterior of a casing, each connector including a ferrule that contains a tip of a single-mode optical fiber, the adapter comprising:
a housing including two opposing connector connecting portions;
a sleeve disposed between the two connector connecting portions and configured so that when the connectors are respectively connected to the two connector connecting portions, the ferrule of each of the connectors is inserted into the sleeve, and the single-mode optical fibers of the connectors are optically connected to each other; and
a dust protection member disposed at a casing side of the sleeve and configured to regulate rotation of the sleeve relative to the housing and to shield an interval between the interior of the casing and an interior of the housing when the connector at the casing side is connected to the connector connecting portion at the casing side.

When the connector at the casing side is connected to the connector connecting portion, the dust protection member preferably contacts a portion of the ferrule and covers an entire circumference of the sleeve.

The dust protection member is preferably made from an elastic body.

By connecting the connector at the casing side, the dust protection member may function to regulate the rotation of the sleeve relative to the housing and to shield the interval between the interior of the casing and the interior of the housing.

The laser light source may be a light source emitting visible light.

A photodetector configured to monitor a connection efficiency between the connectors connected to the two connector connecting portions of the housing is preferably further included.

An endoscope apparatus according to this disclosure comprises:
a casing containing or connected to a laser light source;
   a scope configured to irradiate an object with laser light output from the casing and to receive signal light from the object;
   an image processor configured to generate an image based on the signal light received by the scope; and
   an optical fiber connection adapter that is disposed between the casing and the scope, connects connectors, and guides light from the laser light source from an interior to an exterior of the casing, each connector including a ferrule that contains a tip of a single-mode optical fiber;
   wherein the adapter comprises:
      a housing including two opposing connector connecting portions;
      a sleeve disposed between the two connector connecting portions and configured so that when the connectors are respectively connected to the two connector connecting portions, the ferrule of each of the connectors is inserted into the sleeve, and the single-mode optical fibers of the connectors are optically connected to each other; and
      a dust protection member disposed at a casing side of the sleeve and configured to regulate rotation of the sleeve relative to the housing and to shield an interval between the interior of the casing and an interior of the housing when the connector at the casing side is connected to the connector connecting portion at the casing side.

### (Advantageous Effects of Invention)

According to this disclosure, a dust protection member is disposed at a casing side of the sleeve and is configured to regulate rotation of the sleeve relative to the housing and to shield an interval between the interior of the casing and the interior of the housing when the connector is connected to the connector connecting portion at the casing interior side. Hence, a stable optical fiber connection efficiency can be obtained, and dust can be prevented from entering the adapter housing, thus easing the burden of cleaning the fiber end faces.

### BRIEF DESCRIPTION OF THE DRAWINGS

In the accompanying drawings:
FIG. 1 is a top view of an adapter and connectors according to Embodiment 1;
FIG. 2 is a vertical cross-sectional view of the adapter and connectors of FIG. 1;
FIG. 3 is a vertical cross-sectional view illustrating the adapter and connectors of FIG. 1 in a state of being joined;
FIG. 4A illustrates the positional relationship between the split sleeve and the dust protection ring, and FIG. 4B illustrates the positional relationship between the split sleeve, the dust protection ring, and the inner cylinder of the adapter;
FIG. 5A illustrates the structure of the split sleeve and the inner cylinder of the adapter in Modification 1, and FIG. 5B illustrates the state in which the dust protection ring is embedded in FIG. 5A;
FIG. 6A illustrates the structure of the split sleeve and the inner cylinder of the adapter in Modification 2, FIG. 6B illustrates the shape of the dust protection ring that is embedded in the adapter of FIG. 6A, and FIG. 6C illustrates the state in which the dust protection ring is embedded in the adapter of FIG. 6A;
FIG. 7 is a vertical cross-sectional view of an adapter and connectors according to Embodiment 2;
FIG. 8 is a vertical cross-sectional view illustrating the adapter and connectors of FIG. 7 in a state of being joined;
FIG. 9 is an external view schematically illustrating an endoscope apparatus into which the adapter of this disclosure is integrated; and
FIG. 10 is a block diagram schematically illustrating the structure of the endoscope apparatus of FIG. 9.

### DETAILED DESCRIPTION

Embodiments are described below with reference to the drawings.

### Embodiment 1

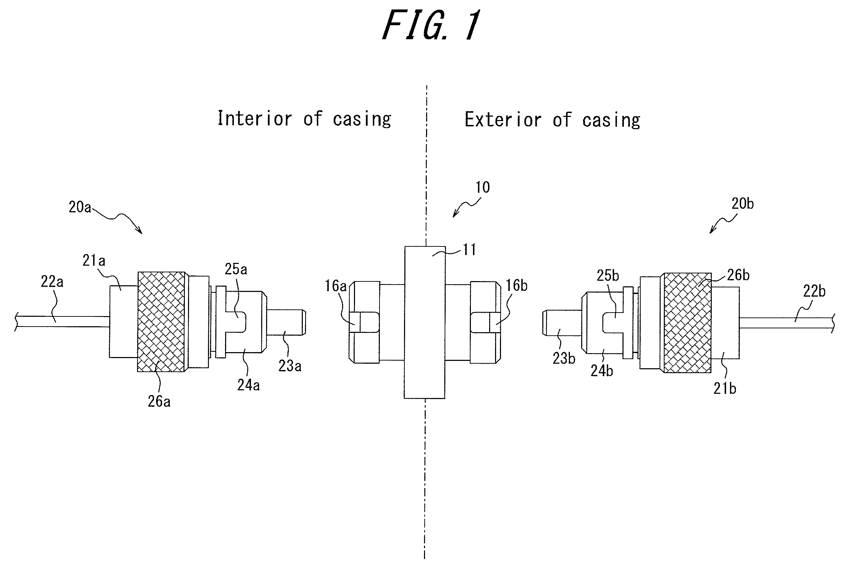

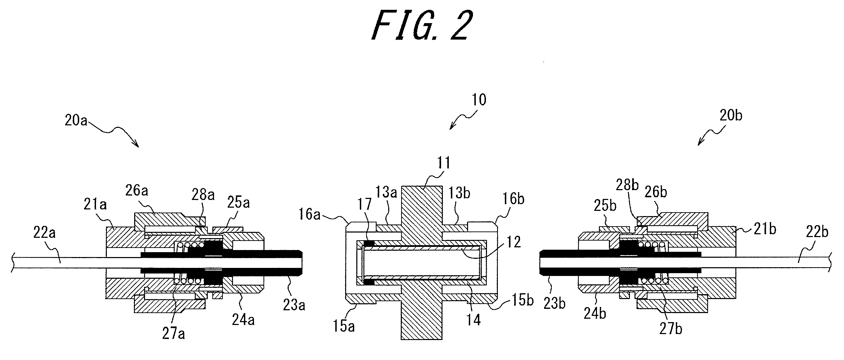

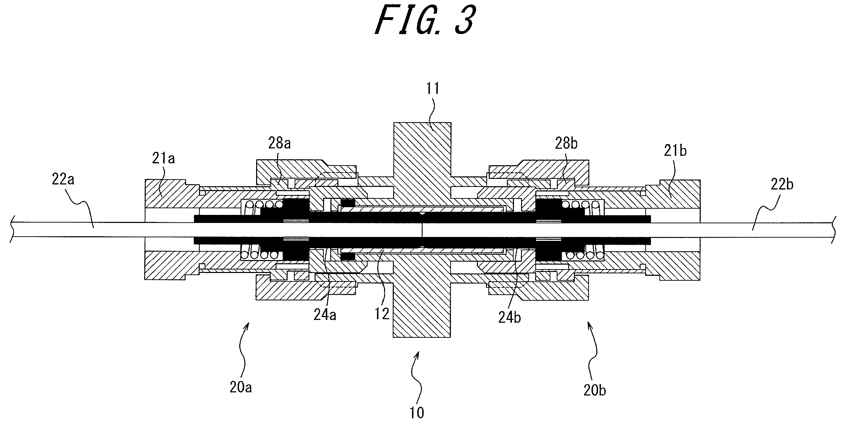

With reference to FIGs. 1 to 3, the adapter and connectors according to Embodiment 1 are described. FIG. 1 is a top view of an adapter 10 and connectors 20a, 20b according to Embodiment 1. FIG. 2 is a vertical cross-sectional view of the adapter 10 and connectors 20a, 20b of FIG. 1. FIG. 3 is a vertical cross-sectional view illustrating the adapter 10 and connectors 20a, 20b of FIG. 1 in a state of being joined.

The adapter 10 of this disclosure is an optical fiber connection adapter between the interior of a casing that contains a laser light source, or of a casing to which a laser light source is connected, and the exterior of the casing. The adapter 10 connects connectors 20a, 20b including ferrules 23a, 23b that contain the tip of respective single-mode optical fibers 22a, 22b. The adapter 10 is disposed at the side of the casing and connects the connector 20a inside the casing to the connector 20b outside the casing.

As illustrated in FIG. 2, the adapter 10 is provided with an adapter housing 11 and a split sleeve 12. The adapter housing 11 includes an outer cylinder 13a that has an opening at the casing interior side and an outer cylinder 13b that has an opening at the casing exterior side. On the inside of the outer cylinders 13a, 13b, the adapter housing 11 includes an inner cylinder 14 that has a hollow space between the connector 20a side and the connector 20b side. The cylindrical split sleeve 12 is disposed in the hollow space of the inner cylinder 14. In order to prevent the split sleeve 12 from becoming detached, the inner circumferential surface at both ends of the inner cylinder 14 projects inward. Furthermore, external screws 15a, 15b are provided at the outer circumferential ends of the outer cylinders 13a, 13b. Furthermore, groove-shaped keyways 16a, 16b are provided at portions of the inner circumferential surface of the outer cylinders 13a, 13b. In this way, two connector connecting portions disposed opposite each other and having a shape that allows connection of the connectors 20a and 20b are formed on the casing interior side and the casing exterior side of the adapter housing 11.

The split sleeve 12 is a hollow tubular member that has a split extending in the longitudinal direction (along the central axis when disposed within the inner cylinder 14) and is formed from a hard ceramic or the like, such as zirconia. Between the split sleeve 12 and the inner cylinder 14 at the connector 20a side inside the housing, a dust protection ring 17 (shielding member) is provided along the outer circumference of the split sleeve 12. The dust protection ring 17 shields the interior of the housing from the interior of the inner cylinder 14 of the adapter housing 11 and is, for example, made of a material such as highly elastic rubber. The dust protection ring 17 is designed so that ultraviolet light from the outside does not reach the dust protection ring 17 due to light being blocked by the adapter housing 11, the casing, and the like. Degradation of the dust protection ring 17 is thus prevented.

The connector 20a includes a connector housing 21 a and the ferrule 23a in which the tip of the single-mode optical fiber 22a is contained. Hereinafter, the tip direction of the single-mode optical fiber 22a in the connector 20a is referred to as "forward", and the opposite direction is referred to as "backward".

The tip of the connector housing 21 a is a cylinder 24a that has a cylindrical wall and has a shape that fits in the space between the inner cylinder 14 of the adapter 10 and the outer cylinder 13a. A key 25a projects from the outer circumferential surface of the cylinder 24a. When connecting the adapter 10 and the connector 20a, the key 25a is inserted into and engages with the keyway 16a of the adapter 10, thereby accurately positioning the adapter 10 and the connector 20a in the direction of rotation.

A coupling nut 26a, which can rotate and can be displaced along the optical fiber axis over a specific range, is provided on the outer circumference of the connector housing 21 a. An internal screw is provided on the inner surface of the coupling nut 26a and engages with the external screw 15a on the outer cylinder 13 a of the adapter housing 11.

The ferrule 23a has a columnar shape with a chamfered tip. The single-mode optical fiber 22a is inserted therein along the central axis of the ferrule 23a. The columnar portion of the ferrule 23a projects forward from the center of the cylinder 24a of the connector housing 21 a, and the outer circumference of the columnar portion is supported by the connector housing 21 a at the back of the cylinder 24a. Furthermore, at the back along the ferrule 23a, a flange is provided. The flange can slide along the inner circumferential surface of the adapter housing 11 over a specific range in the optical axis direction of the single-mode optical fiber 22a within the adapter housing 11 and is biased forward by a spring 27a disposed inside the adapter housing 11.

The connector 20a disposed inside the housing has been described, and the connector 20b outside the housing has a similar structure. Whereas the connector 20a inside the housing is basically maintained in a state of connection over a long period of time, the external connector 20b is removed more frequently than the connector 20a.

With the above-described configuration, when the connectors 20a, 20b are connected to the adapter 10, first the axis of each tip of the adapter 10 is aligned with the axis of the tip of one of the connectors 20a, 20b, and the keys 25a, 25b of the connectors 20a, 20b determine the position in the direction of rotation that allows insertion into the keyways 16a, 16b of the adapter 10. The ferrules 23a, 23b are then inserted into the split sleeve 12, and the cylinders 24a, 24b of the connectors 20a, 20b are inserted between the ends of the outer cylinders 13a, 13b and the inner cylinder 14 of the adapter 10.

Next, the coupling nuts 26a, 26b are moved to the adapter 10 side and rotated. As a result, the external screw 15a of the adapter housing 11 engages with the internal screw of the coupling nut 26a, and the coupling nuts 26a, 26b move forward towards the adapter 10. The ferrule 23a thus slides further forward within the split sleeve 12.

Once the tip of the ferrule 23a of the connector 20a at the casing interior side and the tip of the ferrule 23b of the connector 20b at the casing exterior side abut, the ferrules 23a, 23b are pressed against each other by the spring force of the springs 27a, 27b in the connectors 20a, 20b with a pressure that is at most a certain degree that does not cause damage to the tips of the single-mode optical fibers 22a, 22b. The rotation of the coupling nuts 26a, 26b is stopped by being locked by steps 28a, 28b provided on the outer circumference of the connector housing 21 a, 21 b. As a result, excessive pressure is not generated between the ferrules 23a, 23b.

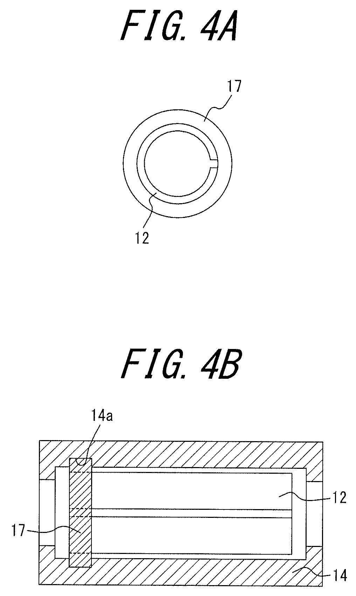

The function of the dust protection ring 17 disposed on the casing side of the adapter 10 is now described further. FIG. 4A illustrates the positional relationship between the split sleeve 12 and the dust protection ring 17, and FIG. 4B illustrates the positional relationship between the split sleeve 12, the dust protection ring 17, and the inner cylinder 14 of the adapter 10. On the inner surface of the inner cylinder 14 of the adapter 10, a recess 14a for containing the dust protection ring 17 is provided. The connector 20a at the casing side where the dust protection ring 17 is disposed is not detached often. Hence, upon the ferrule 23a being inserted into the split sleeve 12, the elastic dust protection ring 17 is pressed between the ferrule 23a and the recess 14a in the inner cylinder 14 of the adapter housing 11. Therefore, the frictional force operating between the split sleeve 12 and the dust protection ring 17, and between the dust protection ring 17 and the inner cylinder 14, inhibits rotation of the split sleeve 12 inside the adapter housing 11. As a result, the angle of rotation of the split sleeve 12 around the optical axis relative to the adapter 10 is regulated.

Rotation of the ferrule 24a is fixed relative to the connector 20a, and the positioning in the direction of rotation between the adapter 10 and the connector 20a is fixed by the key 25a being inserted into the keyway 16a. Hence, by controlling rotation of the split sleeve 12 relative to the adapter 10, the relationship of the angle of rotation between the split sleeve 12 and the ferrule 23a is fixed. The same also holds between the adapter 10 and the ferrule 23b of the connector 20b. If the angular relationship between the split sleeve 12 and the ferrule 23b around the optical axis does not change, variation in the connection efficiency decreases. As a result, the variation in the connection efficiency due to detaching and reattaching the connector 20b to the adapter 10 is reduced.

Furthermore, when the connector 20a is connected to the adapter 10, the dust protection ring 17 shields the interval between the split sleeve 12 and the inner surface of the inner cylinder 14 of the adapter housing 11, thereby shielding the opening at the casing side of the adapter housing 11 from the interior of the inner cylinder 14 of the adapter housing 11. Accordingly, dust can be prevented from entering from the casing side and landing on the end faces of the ferrules 23a, 23b, where the end faces of the single-mode optical fibers 22a, 22b connect.

As described above, according to this embodiment, the dust protection ring 17 is disposed at the casing side of the split sleeve 12, regulates rotation of the split sleeve 12 relative to the adapter housing 11, and shields the interval between the interior of the casing and the interior of the housing when the connector is connected to the connector connecting portion at the casing interior side. Therefore, variation in the connection efficiency of the optical fiber can be reduced, and dust can be prevented from entering the split sleeve 12, thus easing the burden of cleaning the fiber end faces.

### Modification 1

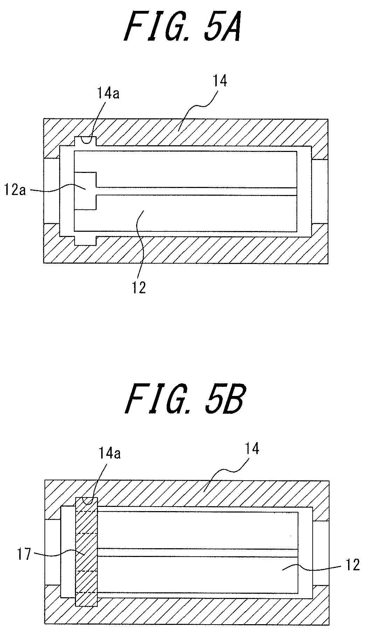

FIG. 5A illustrates the structure of the split sleeve 12 and the inner cylinder 14 of the adapter 10 in Modification 1, and FIG. 5B illustrates the state in which the dust protection ring 17 is embedded in FIG. 5A. In this modification, a larger notch 12a is provided at the split portion of the split sleeve 12, at the location where the dust protection ring 17 of the split sleeve 12 is disposed. As a result, when the ferrule 23a is inserted, the dust protection ring 17 contacts the ferrule 23a at the portion of this notch 12a. In this way, the dust protection ring 17 is in close contact with the ferrule 23a at the portion of the notch 12a and fixes the ferrule 23a. Furthermore, the dust protection ring 17 covers the entire circumference of the split sleeve 12, thereby preventing dust from entering onto the end faces of the ferrules 23a, 23b in the split sleeve 12. A projection with a size that just fits into the portion of the notch 12a of the split sleeve 12 may be provided on the dust protection ring 17. In this way, shielding can be achieved more easily.

### Modification 2

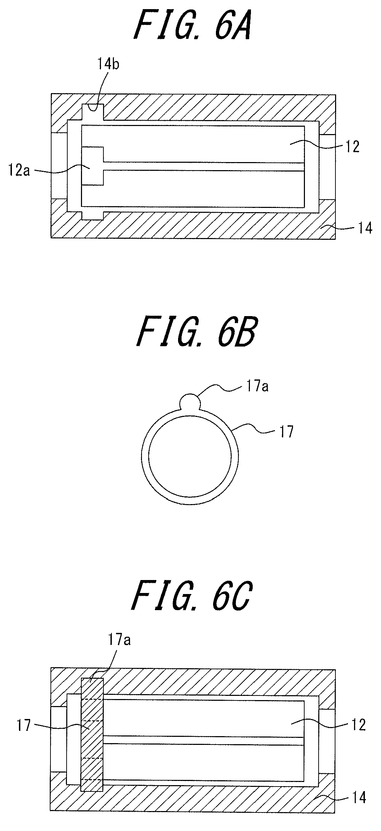

FIG. 6A illustrates the structure of the split sleeve 12 and the inner cylinder 14 of the adapter 10 in Modification 2, FIG. 6B illustrates the shape of the dust protection ring 17 that is embedded in the adapter 10 of FIG. 6A, and FIG. 6C illustrates the state in which the dust protection ring 17 is embedded in FIG. 6A. In the adapter 10 of this modification, a recessed projection receiver 14b is further provided on a portion of the recess 14a on the inner surface of the inner cylinder 14. The dust protection ring 17, which includes a projection 17a, is disposed here. In this modification, as in Modification 1, the dust protection ring 17 is in close contact with the ferrule 23a at the portion of the notch 12a and covers the entire circumference of the split sleeve 12, thereby preventing dust from entering onto the end faces of the ferrules 23a, 23b. Furthermore, the projection 17a of the dust protection ring 17 fits into the projection receiver 14b of the inner cylinder 14 of the adapter housing 11. Hence, the dust protection ring 17 is more completely fixed, and rotation of the split sleeve 12 is also inhibited. Furthermore, the projection 17a regulates the angle relative to the adapter housing 11, thereby increasing the effect of regulating the angle of rotation between the adapter housing 11 and the ferrules 23a, 23b.

### Embodiment 2

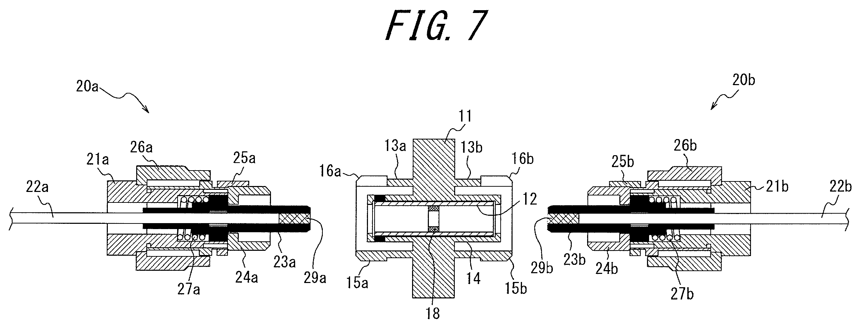

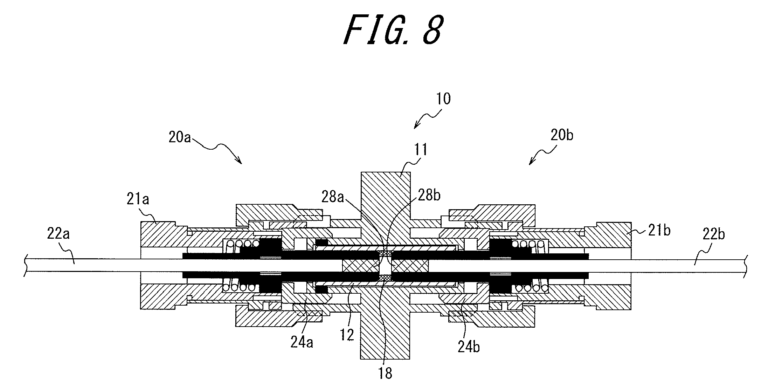

FIG. 7 is a vertical cross-sectional view of an adapter 10 and connectors 20a, 20b according to Embodiment 2, and FIG. 8 is a vertical cross-sectional view illustrating the adapter 10 and connectors 20a, 20b of FIG. 7 in a state of being joined. In this adapter 10, a PD embedded spacer 18 provided with a photodetector (PD) is disposed within the split sleeve 12, in an intermediate portion between the connector 20a side and the connector 20b side. A signal from the photodetector (PD) can be monitored from outside the adapter 10. In the connectors 20a, 20b, collimator lenses 29a, 29b are embedded inside the ferrules 23a, 23b, at the tips of the single-mode optical fibers 22a, 22b. As the collimator lenses 29a, 29b, GRadient INdex (GRIN) lenses having a diameter approximately equivalent to the diameter of the single-mode optical fibers 22a, 22b may be used. Since the remaining structure is similar to that of Embodiment 1, identical structural elements are labeled identically, and a description thereof is omitted.

According to the above structure, by connecting the connectors 20a and 20b to the adapter 10, the ferrule 23a and the ferrule 23b are fixed in the split sleeve 12 with the PD embedded spacer 18 therebetween, as illustrated in FIG. 8. As a result, the single-mode optical fibers 22a, 22b can be connected with a high connection efficiency, without the tips thereof directly abutting. Furthermore, since the tips of the single-mode optical fibers 22a, 22b do not physically come into contact, the risk of damage to the optical fiber tips due to the connectors being connected can be reduced.

The PD embedded spacer 18 is provided with a photodetector, as described above. When the connection efficiency of the single-mode optical fibers 22a and 22b is low, a portion of the light that could not enter the core of the single-mode optical fiber 22b is reflected and strikes the photodetector. Therefore, the connection efficiency between the connectors 20a and 20b can be monitored by detecting the output of the photodetector.

### Embodiment 3

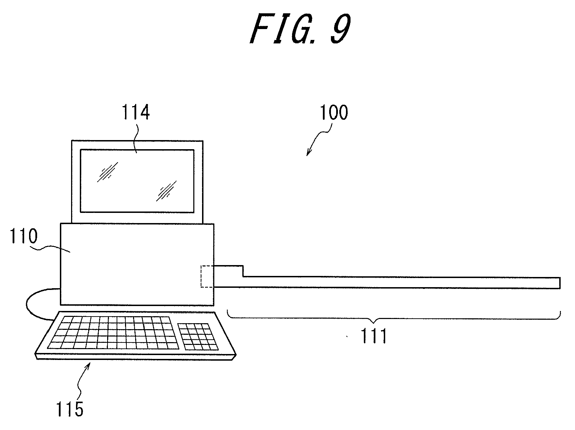

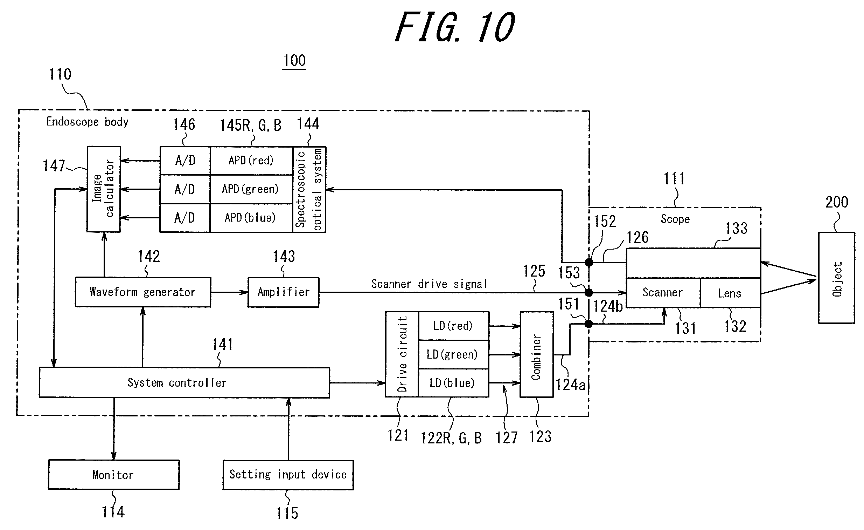

FIG. 9 is an external view schematically illustrating an endoscope apparatus 100 into which the adapter of this disclosure is integrated. FIG. 10 is a block diagram schematically illustrating the structure of the endoscope. apparatus 100 of FIG. 9. The endoscope apparatus 100 includes an endoscope body 110 mounted for example on a dedicated rack stored in an ordinary casing and a scope 111 that is connected detachably to the endoscope body 110. The endoscope body 110 controls the system overall and performs image generation and processing. A dedicated observation monitor 114 and a setting input device 115 for setting observation conditions and the like are connected to the endoscope body 110.

As illustrated in FIG. 10, the endoscope body 110 includes a system controller 141, a drive circuit 121 connected electrically to the system controller 141, LDs (semiconductor lasers) 122R, 122G, 122B that are red, green, and blue semiconductor light sources, an optical fiber type combiner 123, a waveform generator 142, and an amplifier 143. The endoscope body 110 also includes a spectroscopic optical system 144, avalanche photodiodes (APDs) 145R, 145G, 145B that are photodetectors, three A/D converters 146 provided in correspondence with the APDs 145R, 145G, 145B, and an image processor 147.

The illumination light that is laser light emitted by the LDs 122R, 122G, 122B of the endoscope body 110 is input into the combiner 123 by different single-mode fibers 127, combined, and output to a single-mode optical fiber 124a. This single-mode optical fiber 124a is connected to a single-mode optical fiber 124b outside the casing via an optical connection point 151 provided on the casing side of the endoscope body 110. The single-mode optical fiber 124b passes through the scope 111, extending to a position near the tip of the scope 111. The adapter and connectors described in Embodiments 1 and 2 are used for the optical connection point 151.

The endoscope apparatus 100 is a scanning apparatus and is provided with a scanner 131 at the tip of the scope 111. The scanner 131 is a scanning mechanism for scanning a region of observation in an object 200 via a lens 132 with illumination light that has passed through the single-mode optical fiber 124. For example, by supporting the single-mode optical fiber 124, with a magnet connected thereto, at the tip of the scope 111 so as to be swayable, the object 200 may be scanned with a spiral trajectory by applying an oscillating electric field to the single-mode optical fiber 124. Another method for driving the scanner 131 uses a piezoelectric element. The scanning trajectory is not limited to a spiral. A variety of scanning trajectories may be adopted, such as raster scanning or Lissajous scanning.

The drive signal generated by the waveform generator 142 of the endoscope body 110 is provided to the scanner 131 by being amplified by the amplifier 143 and passing through a scanner drive signal line 125 that extends into the scope 111 across an electrical connection point 153 between the endoscope body 110 and the scope 111. As a result, the scanner 131 is controlled by the system controller 141 connected to the waveform generator 142 of the endoscope body 110.

A portion of the light such as reflected light, scattered light, or fluorescent light (detected light) obtained by irradiating the object 200 with illumination light enters a fiber bundle for detection 126 from a fiber bundle incident face for detection 133. The fiber bundle incident face for detection 133 may, for example, be arranged with the incident surface thereof facing the object 200 along the outer periphery of the tip of the scope 111 that faces the object, or may be bundled into a portion of the tip of the scope 111. The fiber bundle for detection 126 is optically connected to a fiber bundle for detection at the endoscope body 110 side by an optical connection point 152 between the endoscope body 110 and the scope 111.

The detected light that propagates to the endoscope body 110 is separated into red, green, and blue components by the spectroscopic optical system 144, and these components are detected by the APDs 145R, 145G, and 145B. The spectroscopic optical system 144 may be configured with a known method such as dichroic mirrors, diffraction elements, color filters, or the like. The red, green, and blue detected light is converted to pixel signals by photoelectric conversion in the APDs 145R, 145G, and 145B, subsequently converted to digital signals by the A/D converters 146, and then transmitted to the image calculator 147.

The image calculator 147 is controlled by the system controller 141 synchronously with the waveform generator 142, associates the successively transmitted red, green, and blue digital pixel signals with the scanning position of illumination light by the scanner 131, and identifies the pixel positions of the pixel signals acquired in chronological order. As a result, each frame of pixel signals is generated consecutively as 2D image data. The generated 2D image data is transmitted to the monitor 114 and displayed. The 2D image data is also stored in a non-illustrated storage device.

In this way, the adapter and connectors of Embodiments 1 and 2 are used at the optical connection point 151 for the single-mode optical fibers between the interior of the endoscope body 110 and the external scope 111. In the endoscope apparatus 100, the scope 111 is detached from the endoscope body 110 for cleaning or the like upon each use, but by using the adapter of Embodiment 1 or Embodiment 2, variation in the connection efficiency due to detachment of the connector can be reduced.

Since the endoscope body 100 includes the light sources LD 22R, 22B, 22G and a plurality of calculation elements, a cooling fan is necessary. Therefore, dust floats inside the casing of the endoscope body 110. This dust enters between the connectors of the single-mode optical fibers to some degree and is a cause of damage to the tip of the single-mode fiber 24. In this embodiment, however, the adapter described in Embodiment 1 or Embodiment 2 is used at the optical connection point 151. Hence, dust inside the endoscope body 110 is prevented from entering into the adapter housing, in particular into the split sleeve.

Therefore, according to this embodiment, variation in the connection efficiency due to detachment of the endoscope body 110 and the scope 111 is controlled to yield a stable connection efficiency, and dust can be prevented from entering the adapter housing, thus easing the burden of cleaning the fiber end faces.

This disclosure is not limited to the above embodiments, and a variety of modifications and changes may be made. For example, in each of the above embodiments, the split sleeve is disposed in the adapter housing so that the ferrule tips abut, but the sleeve does not need to include a split. Furthermore, the dust protection ring does not need to be a circular ring, and a variety of shapes may be adopted. In addition to a shape having a projection as described in the embodiments, the dust protection ring may have a variety of external shapes conforming to the inner shape of the adapter housing and may include a circular hole on the inside for insertion of the split sleeve. Also, the material of the dust protection ring is not limited to rubber. A metallic material (copper, aluminum, or the like) may also be used. For example, metal in a toric shape may be disposed between the inner cylinder of the adapter housing and the sleeve, and when the connector on the casing side is inserted into the sleeve, the metal may be crushed so as to regulate rotation of the sleeve and achieve a dust protection effect.

In the embodiments, the case of applying this disclosure to connectors and an adapter that are FC type, which is a standard in the field of optical communication, has been described, but this disclosure may also be applied with other standards, such as connectors and an adapter that are SC type, ST type, MU type, LC type, and the like. The pair of connectors need not be of the same type, and this disclosure may also be applied to a pair of different connector types. Furthermore, this disclosure may be embodied in a variety of ways by providing, between the adapter and the connectors inserted therein, a function to regulate the relative angle between the ferrules of the connectors and the sleeve of the adapter, a function to press the ferrules into the sleeve, and a function to allow fixing and detachment of the connectors to and from the adapter.

## Claims

1. An optical fiber connection adapter that connects connectors and guides light from a laser light source from an interior to an exterior of a casing, each connector including a ferrule that contains a tip of a single-mode optical fiber, the adapter comprising:
a housing including two opposing connector connecting portions;
a sleeve disposed between the two connector connecting portions and configured so that when the connectors are respectively connected to the two connector connecting portions, the ferrule of each of the connectors is inserted into the sleeve, and the single-mode optical fibers of the connectors are optically connected to each other; and
a dust protection member disposed at a casing side of the sleeve and configured to regulate rotation of the sleeve relative to the housing and to shield an interval between the interior of the casing and an interior of the housing when the connector is connected to the connector connecting portion at the casing side.

2. The adapter of claim 1, wherein when the connector at the casing side is connected to the connector connecting portion, the dust protection member contacts a portion of the ferrule and covers an entire circumference of the sleeve.

3. The adapter of claim 1 or 2, wherein the dust protection member is made from an elastic body.

4. The adapter of claim 1 or 2, wherein by connecting the connector in the casing, the dust protection member functions to regulate the rotation of the sleeve relative to the housing and to shield the interval between the interior of the casing and the interior of the housing.

5. The adapter of any one of claims 1 to 4, wherein the laser light source is a light source emitting visible light.

6. The adapter of any one of claims 1 to 5, further comprising a photodetector configured to monitor a connection efficiency between the connectors connected to the two connector connecting portions of the housing.

7. An endoscope apparatus comprising:
a casing containing or connected to a laser light source;
a scope configured to irradiate an object with laser light output from the casing and to receive signal light from the object;
an image processor configured to generate an image based on the signal light received by the scope; and
an optical fiber connection adapter that is disposed between the casing and the scope, connects connectors, and guides light from the laser light source from an interior to an exterior of the casing, each connector including a ferrule that contains a tip of a single-mode optical fiber;
wherein the adapter comprises:
a housing including two opposing connector connecting portions;
a sleeve disposed between the two connector connecting portions and configured so that when the connectors are respectively connected to the two connector connecting portions, the ferrule of each of the connectors is inserted into the sleeve, and the single-mode optical fibers of the connectors are optically connected to each other; and
a dust protection member disposed at a casing side of the sleeve and configured to regulate rotation of the sleeve relative to the housing and to shield an interval between the interior of the casing and an interior of the housing when the connector at the casing side is connected to the connector connecting portion at the casing side.
